# EUROPEAN PATENT APPLICATION

(11) **EP 2 258 854 A1**
(43) Date of publication of application: **08.12.2010**
(21) Application number: 09160750.7
(22) Date of filing: 20.05.2009
(51) Int. Cl.: C12N 15/67, C12N 15/81

(54) **Eukaryotic host cell comprising an expression enhancer**

(71) Applicant: FH Campus Wien, 1100 Vienna (AT)
(72) Inventor: Stadlmayr, Gerhard, 1100 Vienna (AT); Mattanovich, Diethard, 1120 Vienna (AT); Sauer, Michael, 1040 Vienna (AT)
(74) Representative: Redl, Gerda

(57) **Abstract**

Invention relates to a eukaryotic host cell comprising a recombinant nucleotide sequence encoding an expression enhancer, which is selected from the group consisting of cLC52, RPL33, cLC61 and functional homologues thereof, and its use in a method of producing a protein of interest (POI).

SEQUENCE LISTING

<110>  FH Campus Wien

<120>  Eukaryotic host cell comprising an expression enhancer

<130>  FH1/EP

<160>  25

<170>  PatentIn version 3.3

<210>  1
<211>  633
<212>  DNA
<213>  Pichia pastoris

<400>  1
atgagttttc aagtctacaa catggtgtcc ttgccagcac cgcaagcaac atcattctcc      60

caacatccga aacttccaac attggaagtg atacaacggc caccactcac tggaagacat     120

ggcattcacg aagaagagat caagctacct tcctttagat ctttactggc tagtgcgggc     180

actgccgctg ctcttgaata tccccccagg attcaacccc aatttgcaac aggggcacct     240

gcacctgagc aagcatttta tcagccccta aataccgtgg gagtcacgag gcccaacatt     300

cacacctcca tcactccacc actggaggcg caggacctcc ctcaccaaca aaagtttatg     360

gaaccatttg cgtccttccc caatacagta gtgtcttcta cacccaacac agccatcaga     420

aaatacaagt gcaagatctg cgagaggtct ttcacaacgt cgggccactt agctcgtcac     480

acacgtattc acactggtga aaagaagcat gagtgtcctt tcgaaggctg ttctgctagg     540

ttcagccgtc aggataactg catgcaacat tacaagacgc atgttaatac caagtcgaaa     600

cgaaagagat ctagattgag aagtaaatag gta                                  633

<210>  2
<211>  327
<212>  DNA
<213>  Pichia pastoris

<400>  2
atggctgagg aaaacagatt atacgttaag ggtaagcacg tgtctttcca acgttccaag      60

agtgttatcc acccaaagac ttccttgatc aaaattgagg gtgtcgaaaa ctcaaaggat     120

gctgagttct acattggtaa gagaatcgct tacgtctaca aaggtgtaaa ggctatcaac     180

ggtacaaagg tcagagtcat gtggggaaca gtcaccagaa ctcacggaaa ctctggtgtt     240

gtcagagcta agttcgaacg taaccttcca ggtcagagtt ttggttccac tgtcagaatc     300

atgctttacc catctaacat ctaaatt                                         327

<210>  3
<211>  671
<212>  DNA
<213>  Pichia pastoris

<400>  3
atggctgagg aaaacagatg tatgtactat taaatcgcaa ggtgactggt aaatgatgaa      60

gatgatcggc ttaatgagaa gaataattga aaacatagcc agaaagtttt gatttctgtt     120

aaaccaagct gaaatgatgg gttgggaatt ttttattaag ccaaatgaac attcttacaa 180

ctttaaccaa cggatattat atatggctga gccagcgtta ttccaaaagc acaagtggga 240

acaggtctct aagttttttc tttactttca agttgagttg accgcttcat cttcattttc 300

tccgactatc accaccttct cactatattt tttagattca cgaggttact aactttgcta 360

tagtatacgt taagggtaag cacgtgtctt tccaacgttc caagagtgtt atccacccaa 420

agacttcctt gatcaaaatt gagggtgtcg aaaactcaaa ggatgctgag ttctacattg 480

gtaagagaat cgcttacgtc tacaaaggtg taaaggctat caacggtaca aaggtcagag 540

tcatgtgggg aacagtcacc agaactcacg gaaactctgg tgttgtcaga gctaagttcg 600

aacgtaacct tccaggtcag agttttggtt ccactgtcag aatcatgctt tacccatcta 660

acatctaaat t 671

<210> 4
<211> 621
<212> DNA
<213> Pichia pastoris

<400> 4
atggctccca gaacactacc agaagactta attccctccc tatacgactt gcacatctac 60

aacttccaac ccgaaaaaaa gacttatgat ggagacattg tcatccactt ggaggtgaag 120

gagcccactg atgaagtggt cttcaatgcc aaggatttgg aattgaaaga cgtacatgtc 180

ttccacaatg tcaacaagtc tgaaaacgaa atccccgtta aggagattgt tgataacgag 240

ctcatcacaa ttaagctcaa agagaaggtt acttccggaa cgttgctggt gaatatttcc 300

ttcaccggta acattcaatc tgataaaatt ggattttaca agggagacac agatgtggaa 360

ggaagagtca catacactac aaaccttacc actccaaatg ccaggttggc attcccatgt 420

cttgataaca tattgttgaa agctccattc aagttcggag taactgccaa tccaggacaa 480

ttagtgagtt ccattttgga tctaagctct gaggctgacg tcttgaatga caatgacgat 540

gtgattggta cgagatacca ataccaagtg agtgagccaa tagccccagc tttactggag 600

tggaccattc atatttaacg a 621

<210> 5
<211> 30
<212> DNA
<213> artificial

<220>
<223> primer: 2F5 vH/hc FORW EcoRI

<400> 5
aggagaattc aggatcacgt taaaggaatc 30

<210> 6
<211> 26
<212> DNA
<213> artificial

<220>

<223>  primer: 2F5 vL/lc FORW EcoRI

<400>  6
acaagaattc gccctccaac tgaccc                                          26


<210>  7
<211>  26
<212>  DNA
<213>  artificial

<220>
<223>  primer: cL BACK SacII

<400>  7
ccttccgcgg ctaacactct cccctg                                          26


<210>  8
<211>  32
<212>  DNA
<213>  artificial

<220>
<223>  primer: cH BACK EcoRI

<400>  8
gaattctttg tcacaagatt tgggctcaac tt                                   32


<210>  9
<211>  36
<212>  DNA
<213>  artificial

<220>
<223>  primer: Agg-EcorRI#1

<400>  9
aagaattcac agctagcgcc aaaagctctt ttatct                               36


<210>  10
<211>  30
<212>  DNA
<213>  artificial

<220>
<223>  primer: Agg-XbaI#2

<400>  10
tgttctagat tagaatagca ggtacgacaa                                      30


<210>  11
<211>  55
<212>  DNA
<213>  artificial

<220>
<223>  oligonucleotide: MCS#1

<400>  11
aattcttaat taagcagtta atgcggcgcg ccatgcagcc tatgcttaat taagc          55


<210>  12
<211>  55
<212>  DNA

<213>  artificial

<220>
<223>  oligonucleotide: MCS#2

<400>  12
ggccgcttaa ttaagcatag gctgcatggc gcgccgcatt aactgcttaa ttaag          55


<210>  13
<211>  36
<212>  DNA
<213>  artificial

<220>
<223>  primer: Pgap-back-EcoRI

<400>  13
gtcgtgaatt cctcgtttcg aaatagttgt tcaatt                               36


<210>  14
<211>  41
<212>  DNA
<213>  artificial

<220>
<223>  primer: Pgap-middle-SfiI

<400>  14
aagagcattg ctggccaagg gggccgtaga agaagctctc c                         41


<210>  15
<211>  32
<212>  DNA
<213>  artificial

<220>
<223>  primer: Pgap-for-BglII

<400>  15
tgcatgagat cagatctttt ttgtagaaat gt                                   32


<210>  16
<211>  49
<212>  DNA
<213>  artificial

<220>
<223>  primer: GAPLibamp

<400>  16
accaccagaa tcgaatataa aaggcgaaca cctttcccaa ttttggttt                 49


<210>  17
<211>  50
<212>  DNA
<213>  artificial

<220>
<223>  primer: Shble3out

<400>  17
gcgacccggc cggcaactgc gtgcacttcg tggccgagga gcaggactga                50

<210>  18
<211>  49
<212>  DNA
<213>  artificial

<220>
<223>  primer: RPL33_#1_SbfI

<400>  18
gattcacctg caggccatgg ctgaggaaaa cagattatac gttaagggt                    49


<210>  19
<211>  48
<212>  DNA
<213>  artificial

<220>
<223>  primer: RPL33_#2_SfiI

<400>  19
ggatggccga ggcggcctta gatgttagat gggtaaagca tgattctg                     48


<210>  20
<211>  53
<212>  DNA
<213>  artificial

<220>
<223>  primer: cLC52_#1_SbfI

<400>  20
gattcacctg caggccatga gttttcaagt ctacaacatg gtgtccttgc cag               53


<210>  21
<211>  54
<212>  DNA
<213>  artificial

<220>
<223>  primer: cLC52_#2_SfiI

<400>  21
ggatggccga ggcggcccta tttacttctc aatctagatc tctttcgttt cgac              54


<210>  22
<211>  55
<212>  DNA
<213>  artificial

<220>
<223>  primer: cLC61_#1_SbfI

<400>  22
gattcacctg caggccatgg ctcccagaac actaccagaa gacttaattc cctcc             55


<210>  23
<211>  49
<212>  DNA
<213>  artificial

<220>
<223>  primer: cLC61_#2_SfiI

<400>  23

ggatggccga ggcggcctta aatatgaatg gtccactcca gtaaagctg 49

<210> 24
<211> 29
<212> DNA
<213> artificial

<220>
<223> primer: KanR#1

<400> 24
ccgaggtacc gacatggagg cccagaata 29

<210> 25
<211> 30
<212> DNA
<213> artificial

<220>
<223> primer: KanR#2

<400> 25
ccgaggtacc agtatagcga ccagcattca 30

## Description

The invention relates to a eukaryotic host cell comprising an expression enhancer and its use in a method to produce a protein of interest (POI).

Successful secretion of proteins has been accomplished both with prokaryotic and eukaryotic hosts. The most prominent examples are bacteria like *Escherichia coli,* yeasts like *Saccharomyces cerevisiae, Pichia pastoris* or *Hansenula polymorpha,* filamentous fungi like *Aspergillus awamori* or *Trichoderma reesei,* or mammalian cells like e.g. CHO cells. While the secretion of some proteins is readily achieved at high rates, many other proteins are only secreted at comparatively low levels (Punt et al., 2002; Macauley-Patrick et al., 2005; Porro et al., 2005).

One approach for the improvement of the secretion of a recombinant protein was done by random mutagenesis (Archer et al., 1994; Lang and Looman, 1995). The major disadvantage of this method is that positive results usually cannot be transferred to other strains.

The secretory pathway - the folding and processing of proteins - of eukaryotic organisms, e.g. of yeast, is very complex with many interacting participants. Some of these proteins have catalytic activity on the proteins like protein disulfide isomerase (PDI), others act by binding to the proteins and preventing them from aggregation (chaperones, e.g. BiP), or by stimulating release of the protein to the cell exterior at a later step in the secretory pathway (SSO proteins). Due to this interdependence, increasing the rate of one reaction step in the secretory pathway may not automatically augment secretion of a protein of interest, but instead may cause a rate-limitation at one or more of the subsequent reaction steps and thus may not remove but only shift bottle-neck(s) of the expression system.

It has been shown in several cases that the secretion process of heterologous proteins can be enhanced by co-overexpression of certain proteins which are called helper factors (Mattanovich et al., 2004).

WO 2008/128701A2 describes an expression system to increase the secretion of a POI from a eukaryotic cell employing a secretion helper factor selected from the group consisting of BMH2, BFR2, C0g6, C0Y1, CUP5, IMH 1, KIN2, SEC31, SSA4 and SSE1. The relevant genes were obtained from cDNA micoarrays of S. *cerevisiae.*

Co-expression of the gene encoding PDI and a gene encoding a heterologous disulphide-bonded protein was first suggested in WO 93/25676 as a means of increasing the production of the heterologous protein. WO 93/25676 reports that the recombinant expression of antistasin and tick anticoagulant protein can be increased by co-expression with PDI.

WO 94/08012 provides methods for increasing protein secretion in yeast by increasing expression of a Hsp70 chaperone protein, i.e. KAR2 and BiP or a PDI chaperone protein.

WO 03/057897 provides methods for the recombinant expression of a protein of interest by co-expressing at least two genes encoding proteins selected from the group consisting of the chaperone proteins GroEL, GRoES, Dnak, DnaJ, GRpe, ClpB and homologs thereof.

WO 2005/0617818 and WO 2006/067511 provide methods for producing a desired heterologous protein in yeast by using a 2µm-based expression plasmid. It was demonstrated that the production of a heterologous protein is substantially increased when the genes for one or more chaperone protein(s) and a heterologous protein are co-expressed on the same plasmid.

Another approach to stimulate the secretory pathway is to overexpress the unfolded protein response (UPR) activating transcription factor HAC1. Transcriptional analyses revealed that up to 330 genes are regulated by HAC1, most of them belonging to the functional groups of secretion or the biogenesis of secretory organelles (e.g. ER-resident chaperones, foldases, components of the Translocon).

WO 01/72783 describes methods for increasing the amount of a heterologous protein secreted from a eukaryotic cell by inducing an elevated unfolded protein response (UPR), wherein the UPR is modulated by co-expression of a protein selected from the group consisting of HAC1, PTC2 and IRE1.

While these approaches, once established, can be transferred to other strains and used for other proteins as well, they are limited by the actual knowledge about the function of such proteins supporting the secretion of other proteins.

It can be anticipated that the successful high level secretion of a recombinant protein may be limited at a number of different steps, like folding, disulfide bridge formation, glycosylation, transport within the cell, or release from the cell. Many of these processes are still not fully understood. Such helper functions cannot be predicted with the current knowledge of the state-of-the-art, even when the DNA sequence of the entire genome of a host organism is available.

Wentz and Shusta (Appl. Environ. Microbiol. (2007) 73(4): 1189-1198) employ a *Saccharamyces cerevisiae* yeast surface display gene library to identify improved secretion strains using an appropriate selection pressure. The yeast cDNAs enhancing scTCR display were CCW12, SEDI, CWP2, RPP0, which were found to be temperature dependent, and ERO1 that was found to be a lone 20°C enhancer.

It is an object of the invention to provide new methods to increase production of secreted proteins in eukaryotic cells, in particular in yeast cells of the Pichia genus, which are simple and efficient. It is desirable to provide new genes to be used in methods for the increased production of secreted proteins by overexpression of helper factors that could be employed in industrial production methods.

It is a further object of the present invention to provide a recombinant host cell with improved properties to increase production of a POI.

The object is solved by the subject matter as claimed.

In particular, the invention refers to a eukaryotic host cell comprising a recombinant nucleotide sequence encoding an expression enhancer, which is selected from the group consisting of cLC52, RPL33, cLC61 and functional homologues thereof.

The host cell according to the invention preferably comprises the nucleotide sequence, which is originating from *P. pastoris.*

The host cell according to the invention preferably is a fungal cell, more preferably a yeast cell, or a higher eukaryotic cell, preferably a mammalian or a plant cell.

According to a specific aspect of the invention the host cell is a cell of the Pichia genus, in particular a cell of a strain of *P. pastoris.*

In one aspect the invention relates to such a host cell, which is a wildcard host cell for the introduction of a gene of interest encoding a POI.

In another aspect the invention relates to such a host cell, which is a producer cell comprising a recombinant nucleotide sequence encoding a POI, capable of producing the POI.

The invention preferably provides for a *P. pastoris* host cell comprising
- a recombinant nucleotide sequence encoding an expression enhancer, which is selected from the group consisting of cLC52, RPL33 and cLC61, and
- a gene of interest encoding a POI.

Such a nucleotide sequence encoding a protein selected from the group consisting of cLC52, RPL33 cLC61 and functional homologues thereof, preferably is used according to the invention as an expression enhancer to increase the expression of a POI in a eukaryotic host cell.

The invention further provides for a method of producing a producer host cell, comprising
- providing a wildcard host cell according to the invention that comprises the recombinant nucleotide sequence encoding a protein selected from the group consisting of cLC52, RPL33 cLC61 and functional homologues thereof, and
- introducing a nucleotide sequence encoding a POI.
   According to an alternative embodiment, the method to produce a producer host cell comprises the steps of
- providing a eukaryotic host cell,
- introducing a nucleotide sequence encoding at least one of cLC52, RPL33, cLC61 and functional homologues thereof, and
- introducing a nucleotide sequence encoding a POI.
   Thus, the invention provides for a method of producing a POI in a eukaryotic cell, comprising the steps of
- providing a producer host cell according to claim 6,
- co-expressing the POI and the expression enhancer in a cell culture, and
- obtaining the POI from the cell culture.

In a preferred method according to the invention the POI is a polypeptide, preferably a eukaryotic polypeptide, selected from the group consisting of serum proteins, such as an immunoglobulin or serum albumin, enzymes, hormones, signalling molecules, matrix proteins, fragments or derivatives thereof.

In another preferred method the POI is mediating the production of a metabolite of the host cell, preferably the POI is a substrate, an enzyme or a cofactor, and a metabolite is produced by the host to obtain a sufficient yield of the metabolite.

Preferably the recombinant nucleotide sequence encoding a POI is provided on a plasmid suitable for integration into the genome of the host cell or for autonomous replication in the host cell.

It is further preferred that the plasmid is a eukaryotic expression vector, preferably a yeast expression vector comprising a secretion leader sequence effective to cause secretion of the POI from the host cell and/or a promoter sequence effective to cause expression of the POI in the host cell.

In one aspect the invention relates to a co-overexpression plasmid for use in a method according to the invention comprising
- the recombinant nucleotide sequence encoding a POI, and
- a nucleotide sequence encoding a protein selected from the group consisting of cLC52, RPL33 cLC61 and functional homologues thereof.

### DETAILED DESCRIPTION OF THE INVENTION

"Biologically active fragment" of a protein shall mean a fragment of a protein that exerts a biological effect similar or comparable to the full length protein. Such fragments can be produced e.g. by amino- and carboxy- terminal deletions as well as by internal deletions.

The term "derivative" encompasses any combination of one or more compounds, e.g. polypeptides or proteins, and / or a fusion compound, in which any part of the compound may be fused at any position to one or more other polypeptides. A derivative may also be obtained by association or binding to other substances by various chemical techniques such as covalent coupling, electrostatic interaction, disulphide bonding etc. The other substances bound to the compound may be lipids, carbohydrates, nucleic acids, organic and inorganic molecules or any combination thereof (e.g. PEG, prodrugs or drugs). The term "derivative" would also comprise a homologous compound, also potentially containing non-natural or chemically modified amino acids, and fragments of compounds. It is understood that the terms cLC52, RPL33 or cLC61 also refer to derivatives thereof, which may by functional equivalents , and/or have similar or improved functions.

"Expression vectors" as used herein are defined as DNA sequences that are required for the transcription of cloned recombinant nucleotide sequences, i.e. of recombinant genes and the translation of their mRNA in a suitable host organism. Such expression vectors usually comprise an origin for autonomous replication in the host cells, selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together.

The term "eukaryotic host" shall mean any eukaryotic cell or organism, which may be cultivated to express a POI. It is well understood that the term does not include human beings.

The term "functionally equivalent variant" of a polypeptide or a nucleotide sequence as used herein means a sequence resulting from modification of this sequence by insertion, deletion or substitution of one or more amino acids or nucleotides within the sequence or at either or both of the distal ends of the sequence, and which modification does not affect (in particular impair) the activity of this sequence.

The term "gene product" or "product of a gene" is the biochemical material, either RNA or protein, resulting from expression of a gene, such as the cLC52, RPL33 or cLC61 genes.

As used herein, a "homologue" or "functional homologue" of a polypeptide shall mean that polypeptides have the same or conserved residues at a corresponding position in their primary, secondary or tertiary structure. The term also extends to two or more nucleotide sequences encoding homologous polypeptides. In particular, homologous compounds usually have at least about 50% amino acid sequence identity with regard to a full-length native sequence or any fragment thereof. Preferably, a homologous compound will have at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native compound, or any other specifically defined fragment of a full-length compound. When the function as a helper factor is proven with such a homologue, the homologue is called "functional homologue".

The term "homologous nucleotide sequences" as used herein refers to nucleotide sequences which are related but not identical in their nucleotide sequence with the contemplated nucleotide sequence, and perform essentially the same function. These are also meant to encompass variations in its nucleotide composition including variations due to the degeneracy of the genetic code, whereby the nucleotide sequence performs essentially the same function.

The term "host cell" or "hosts cell line" refers to a microorganism, used for expression of a recombinant gene to produce polypeptides or metabolites mediated by such polypeptides. A host cell clone of cultivated host cells that have proliferated is commonly understood to be a host cell line. A production host cell line is commonly understood to be a cell line ready-to-use for cultivation in a bioreactor to obtain the gene product in a production method.

The term "operably linked" as used herein refers to the association of nucleotide sequences on a single nucleic acid molecule, e.g. a vector, in a way such that the function of one or more nucleotide sequences is affected by at least one other nucleotide sequence present on said nucleic acid molecule. For example, a promoter is operably linked with a coding sequence of a recombinant gene, when it is capable of effecting the expression of that coding sequence.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The terms "plasmid" and "vector" as used herein include autonomously replicating nucleotide sequences as well as genome integrating nucleotide sequences.

The term "polypeptide" refers to a protein or peptide that contains two or more amino acids, typically at least 3, preferably at least 20, more preferred at least 30, more preferred at least 50 amino acids. The term also refers to higher molecular weight polypeptides, such as proteins. Hereinafter the terms "polypeptide" and "protein" are interchangeably used.

"Promoter" as used herein refers to a DNA sequence capable of controlling the expression of a coding sequence or functional RNA. Promoter activity may be assessed by its transcriptional efficiency. This may be determined directly by measurement of the amount of mRNA transcription from the promoter, e.g. by Northern Blotting or indirectly by measurement of the amount of gene product expressed from the promoter.

The term "protein of interest (POI)" as used herein refers to a protein that is produced by means of recombinant technology in a host cell. More specifically, the protein may either be a polypeptide not naturally occurring in the host cell, i.e. a heterologous protein, or else may be native to the host cell, i.e. a homologous protein to the host cell, but is produced, for example, by transformation with a self replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, e.g. of the promoter sequence.

The term "cLC52" shall mean a polypeptide of *P. pastoris* with homology to transcriptional regulators. In *S. cerevisiae,* the proteins with highest homology to cLC52 are Nrg1 and Nrg2, transcriptional repressors mediating glucose repression. Respective external IDs are: SGD YDR043C, SGD YBR066C.

The term "RPL33" shall mean a polypeptide of *P. pastoris* encoded by a rRNA gene with homology to S. cerevisiae RPL33A and RPL33B (Ribosomal protein L37 of the large (60S) ribosomal subunit). Respective external IDs are: SGD YPL143W and YDR234C. The term shall encompass the full-length protein or biologically active fragments thereof.

The term "cLC61" shall mean a polypeptide of *P. pastoris* encoded by a gene which is homologous to peptidases.

As used herein the terms "cLC52, RPL33 or cLC61" are used interchangeably for the genes and the gene products. Sequence informations to these polypeptides are provided below. For the purpose of this invention, these factors are also called helper factors. The terms shall encompass the full-length protein or biologically active fragments thereof, as well as functional homologues.

It was surprising to obtain such genes and gene products that could be used as protein expression and/or secretion, which have not been characterized as functional helper factor so far. It was found that in this regard relevant genes were cLC52, RPL33 and cLC61, which - upon co-expression could increase the yield in protein production. These genes have functions that would not assumably have been related to a POI expression and/or secretion.

To prove the helper function of the relevant genes, a cDNA library derived from different strains of *P. pastoris,* grown in a wide range of cultivation condition was co-overexpressed in a strain of *P. pastoris* displaying the Fab fragment of the monoclonal antibody on the cell surface. Strains with an increased fluorescence signal after immunfluorescence staining were enriched by using high throughput fluorescence activated cell sorting. After several rounds of culturing and sorting, the genes RPL33, cLC52 and cLC61 could be identified, acting positively on the amount of the displayed Fab fragment. A more detailed description of the experimental procedure can be found in Example 1. The identified genes were amplified by PCR from *P. pastoris* cDNA and cloned into a *P. pastoris* expression vector and transformed in strains of *P. pastoris* pre-selected for high level production of various different POI, either surface-displayed or secreted. To estimate the effect of the co-overexpressed gene on the amount of surface-displayed or secreted recombinant POI, the obtained *P. pastoris* strains according to the invention may be cultured in shake flask experiments and fedbatch or chemostat fermentations in comparison with strains only expressing the POI. All three genes result in an increased cell surface signal in a co-overexpression study. In particular, co-overexpressed cLC52 significantly increased the amount of secreted POI.

According to preferred mode of the invention the recombinant nucleotide sequence comprises the cLC52 gene, which is Seq. ID No. 1, or functional homologues thereof.

According to another preferred mode of the invention the recombinant nucleotide sequence comprises the RPL33 gene, which is Seq. ID No. 2 and 3, respectively, or functional homologues thereof.

According to another preferred mode of the invention the recombinant nucleotide sequence comprises the cLC61 gene, which is Seq. ID No. 4, or functional homologues thereof.

By means of the inventive helper factors the method according to the invention preferably not only refers to an increased production by an enhanced expression, but also to an increased secretion of the POI in a eukaryotic cell. An increase in secretion of the POI is determined on the basis of a comparison of its secretion yield in the presence or absence of co-expression of a said protein that increases protein secretion.

The POI can be any eukaryotic or prokaryotic polypeptide. It can be a naturally secreted protein or an intracellular protein, i.e. a protein which is not naturally secreted. The present invention also provides for the recombinant production of functional homologues, functional equivalent variants, derivatives and biologically active fragments of naturally secreted or not naturally secreted proteins. Functional homologues are preferably identical with or correspond to and have the functional characteristics of a sequence

A secreted POI referred to herein may be, but is not limited to, a protein suitable as a biopharmaceutical substance like an antibody or antibody fragment, growth factor, hormone, enzyme, vaccine, or a protein which can be used for industrial application like e.g. an enzyme.

The POI is preferably a heterologous recombinant polypeptide or protein, which may advantageously be produced in a eukaryotic cell, preferably a yeast cell, preferably as secreted proteins. Examples of preferably produced proteins are immunoglobulins, aprotinin, tissue factor pathway inhibitor or other protease inhibitors, and insulin or insulin precursors, insulin analogues, growth hormones, interleukins, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor1, serum albumin, enzymes, such as lipases or proteases, or a functional analogue of any one of these proteins. In the present context, the term "functional analogue" is meant to indicate a polypeptide with a similar function as the native protein. The polypeptide may be structurally similar to the native protein and may be derived from the native protein by addition of one or more amino acids to either or both the C- and N-terminal end or the side-chain of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

In another embodiment, the POI is a eukaryotic protein or a biologically active fragment thereof, preferably an immunoglobulin or an immunoglobulin fragment such as a Fc fragment or a Fab fragment. Most preferably, the POI is a Fab fragment of the monoclonal anti-HIV1 antibody 2F5.

A POI can also be selected from substrates, enzymes, inhibitors or cofactors that provide for biochemical reactions in the host cell, with the aim to obtain the product of said biochemical reaction or a cascade of several reactions (e.g. a metabolite of the host cell). Examplary products can be vitamins, such as riboflavin, organic acids, and alcohols, which can be obtained with increased yields following the expression of a POI according to the invention..

In general, the host cell, from which the proteins are secreted can be any eukaryotic cell suitable for recombinant expression of a POI.

Examples of preferred yeast cells used as host cells according to the invention include but are not limited to the *Saccharomyces* genus (e.g. *Saccharomyces cerevisiae)*, the *Pichia* genus (e.g. *P. pastoris, or P. methanolica*), the *Komagataella* genus (*K. pastoris, K. pseudopastoris* or *K. phaffii*), *Hansenula polymorpha* or *Kluyveromyces lactis.*

Newer literature divides and renames *Pichia pastoris* into *Komagataella pastoris, Komagataella phaffii* and *Komagataella pseudopastoris* (Kurtzman, 2005). Herein *Pichia pastoris* is used synonymously for all, *Komagataella pastoris, Komagataella phaffii* and *Komagataella pseudopastoris*.

The yeast producer organism preferably used according to the invention may be any suitable yeast organism which, on cultivation, produces large amounts of the heterologous protein or polypeptide in question. Preferred examples of suitable yeast organisms are strains selected from the yeast species *Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida* sp., *Candida utilis, Candida cacaoi, Geotrichum* sp., and *Geotrichum fermentans.*

The most preferred yeast host cells are derived from methylotrophic yeast, such as from Pichia or Komagataella, e.g. Pichia pastoris, or Komoagataella pastoris, or K. phaffii, or K. pseudopastoris. Examples of the host include yeasts such as P. pastoris. Examples of *P. pastoris* strains include CBS 704 (=NRRL Y-1603 = DSMZ 70382), CBS 2612 (=NRRL Y-7556), CBS 7435 (=NRRL Y-11430), CBS 9173-9189, and DSMZ 70877 (German Collection of Microorganisms and Cell Cultures), examples of *S. cerevisiae* strains include W303, CEN.PK and the BY-series (EUROSCARF collection). All of the strains described above have been successfully used to produce transformants and express heterologous genes.

In general, the proteins of interest referred to herein may be produced by methods of recombinant expression well known to a person skilled in the art.

It is understood that the methods disclosed herein may further include cultivating said recombinant host cells under conditions permitting the expression of the POI. A secreted, recombinantly produced POI can then be isolated from the cell culture medium and further purified by techniques well known to a person skilled in the art.

The nucleotide sequences encoding the proteins that increase protein secretion can be obtained from a variety of sources. The origin of a recombinant helper nucleotide sequence (also called "helper gene") according to the invention is preferably from a plant, insect, fungal or bacterial species, preferably from yeast, more preferably from a yeast strain different from the host yeast strain to obtain a recombinant strain. A preferred Pichia host cell according to the invention, such as a *P. pastoris* host cell, contains the heterologous helper factor genes, which are preferably derived from a S. *cerevisiae* strain or another *P. pastoris* strain, different from the production host. In another specific embodiment the host cell according to the invention comprises a recombinant nucleotide sequence with the helper factor genes originating from the same strain as the host cell.

According to a specific embodiment of the invention a helper gene preferably is obtained from a source cell of the same genus as the production host cell. For example, a nucleotide sequence encoding one of the expression helper factor according to the invention may be derived from yeast, such as a *S.cerevisiae* strain, and be used to co-express the helper factor with a POI in a yeast, such as a *S.cerevisiae* producer host cell line. A specifically preferred embodiment relates to a recombinant nucleotide sequence encoding a helper factor originating from *P.pastoris* for use in a method to co-express the helper factor with the POI in a *P.pastoris* producer host cell line. The homologous origin of the nucleotide sequence facilitates its incorporation into the host cell of the same genus, thus enabling stable production of a POI possibly with increased yields in industrial manufacturing processes. Also, functional equivalent nucleotide sequences from other suitable yeasts or other fungi or from other organisms such as vertebrates can be used.

In another preferred embodiment of the invention a helper factor nucleotide sequence is used that is isolated from *Saccharomyces cerevisiae,* particularly as an enhancer of the secretion of a POI in a eukaryotic cell, preferably in a yeast cell and most preferred in a cell of a strain of *P. pastoris.*

According to the invention it is also possible to provide a wildcard host cell according to the invention, which is comprising at least one of the helper factor genes, and which is ready to incorporate a gene of interest encoding a POI. The wildcard cell line is thus a preformed host cell line, which is characerized for its expression capacity. This follows an innovative "wildcard" strategy for the generation of producer cell lines (also called expression host cell line) for the production of biopharmaceuticals, e.g. using site-specific recombinase-mediated cassette exchange. Such a new host cell facilitates the cloning of a GOI, e.g. into predetermined genomic expression hot spots within days in order to get reproducible, highly efficient production cell-lines.

According to the invention it is preferred to provide a *P. pastoris* host comprising the helper factor gene operably linked to the nucleotide sequence codening for the POI.

According to a preferred embodiment the method of the invention employs a recombinant nucleotide sequence encoding the POI, which is provided on a plasmid suitable for integration into the genome of the host cell, in a single copy or in multiple copies per cell. The recombinant nucleotide sequence encoding the POI may also be provided on an autonomously replicating plasmid in a single copy or in multiple copies per cell.

Alternatively, the recombinant nucleotide sequence encoding the POI and the recombinant nucleotide sequence encoding a protein that increases protein secretion are present on the same plasmid in single copy or multiple copies per cell.

The preferred method according to the invention employs a plasmid, which is a eukaryotic expression vector, preferably a yeast expression vector. Expression vectors may include but are not limited to cloning vectors, modified cloning vectors and specifically designed plasmids. The preferred expression vector as used in the invention may be any expression vector suitable for expression of a recombinant gene in a host cell and is selected depending on the host organism. The recombinant expression vector may be any vector which is capable of replicating in or integrating into the genome of the host organisms, also called host vector, such as a yeast vector, which carries a DNA construct according to the invention. A preferred yeast expression vector is for expression in a yeast selected from the group consisting of methylotrophic yeasts represented by the genera Hansenula, Pichia, Candida and Torulopsis.

In the present invention, it is preferred to use a pPICZ, pGAPZ, pPIC9, pPICZalfa, pGAPZalfa, pPIC9K, pGAPHis or pPUZZLE-derived plasmids as the vector.

According to a preferred embodiment of the present invention, a recombinant helper gene, POI gene or recombinant host cell is obtained by ligating the relevant genes into a vector and constructing a single vector carrying the genes, or separate vectors to carry the helper factor genes and the POI genes, respectively. These genes can be stably integrated into the host cell genome by transforming the host cell using such vectors. The polypeptides encoded by the genes can be produced using the recombinant host cell line by culturing a transformant, thus obtained in an appropriate medium, isolating the expressed POI from the culture, and purifying it by a method appropriate for the expressed product, in particular to separate the POI from the co-expressed helper factor.

The relevant genes can be modified to be highly expressed in the host cell according to the invention. The genes can be modified by optimizing the gene sequence to correlate with those codons most frequently used in the host. For example, the gene sequence can be optimized according to the codons used with genes which are highly expressed in the host.

Several different approaches for the POI expression and secretion in the eukaryotic host cell are preferred. Proteins are expressed, processed and secreted by transforming the eukaryotic organism with an expression vector harbouring DNA encoding the desired protein and a signal peptide, preparing a culture of the transformed organism, growing the culture and recovering the protein from the culture medium. The signal peptide may be the desired protein's own signal peptide, a heterologous signal peptide or a hybrid of a native and a heterologous signal peptide. In the present context, the term "signal peptide" is understood to mean a presequence which is present as an N-terminal sequence on the precursor form of an extracellular protein expressed in yeast. The function of the signal peptide is to allow the heterologous protein to be secreted to enter the endoplasmatic reticulum. The signal peptide is normally cleaved off in the course of this process. The signal peptide may be heterologous or homologous to the host organism producing the protein.

In a preferred aspect the invention relates to such a method, wherein the expression vector comprises a secretion leader sequence effective to cause secretion of the POI from the host cell. The presence of such a secretion leader sequence in the expression vector is required when the POI intended for recombinant expression and secretion is a protein which is not naturally secreted and therefore lacks a natural secretion leader sequence, or its nucleotide sequence has been cloned without its natural secretion leader sequence. The secretion leader sequence may originate from yeast source, e.g. from yeast α-factor such as MFα of *Saccharomyces cerevisiae*, or yeast phosphatase, from mammalian or plant source, or others. The selection of the appropriate secretion leader sequence is apparent to a skilled person.

Alternatively, the secretion leader sequence can be fused to the nucleotide sequence encoding a POI intended for recombinant expression by conventional cloning techniques known to a skilled person. In preferred embodiments, the nucleotide sequence of the POI is fused to a secretion signal, i. e. a peptide that targets protein to the secretory pathway where the signal peptide is cleaved and the protein released in the medium, for example alfa-factor, PHO or AGA-2.

Appropriate expression vectors comprise regulatory sequences suitable for expression of DNA encoding a heterologous polypeptide or protein in a eukaryotic host cell. Examples of regulatory sequences include promoters, operators, and enhancers, ribosomal binding sites, and sequences that control transcription and translation initiation and termination. The regulatory sequences may be operably linked to the DNA sequence to be expressed. For example, a promoter sequence is said to be operably linked to a coding sequence, if the promotor controls the transcription of the coding sequence.

The promoter may be any DNA sequence which shows transcriptional activity in the host cell and may be derived from genes encoding proteins either homologous or heterologous to the host. The promoter is preferably derived from a gene encoding a protein homologous to the host cell.

In a further aspect the invention relates to such a method, wherein the expression vector comprises a promoter sequence effective to cause expression of the POI in the host cell. To allow expression of a recombinant nucleotide sequence in a host cell, the expression vector may provide the recombinant nucleotide sequence with a functional promoter adjacent to the 5' end of the coding sequence. The transcription is thereby regulated and initiated by this promoter sequence.

Suitable promoter sequences for use with mammalian host cells may include but are not limited to promoters obtained from the genomes of viruses, heterologous mammalian promoters, e.g. the actin promoter or an immunoglobulin promoter, and heat shock protein promoters.

Further suitable promoter sequences for use with yeast host cells may include but are not limited to promoters obtained from genes that code for metabolic enzymes which are known to be present at high concentration in the cell, e.g. glycolytic enzymes like triosephosphate isomerase (TPI), phosphoglycerate kinase (PGK), glyceraldehyde-3-phosphate dehydrogenase (GAPDH), alcohol oxidase (AOX), lactase (LAC) and galactosidase (GAL).

Preferred examples of suitable promoters are the yeast promoters, which contains a DNA sequence that functions as a promoter for gene transcription in yeast cells. Preferred examples are S. *cerevisiae* Mal, TPI, CUP, ADH or PGK promoters, or the *P. pastoris* glucose-6-phosphate isomerase promoter (PPGI), the 3-phosphoglycerate kinase promoter (PPGK) or glycerol aldehyde phosphate dehydrogenase promoter PGAP, the alcohol oxidase promoter (PAOX), formaldehyde dehydrogenase promoter (PFLD), isocitrate lyase promoter(PICL), translation elongation factor promoter (PTEF), and the promoters of P. pastoris enolase 1 (PENO1), triose phosphate isomerase (PTPI), alpha-ketoisocaproate decarboxylase (PTHI), ribosomal subunit proteins (PRPS2, PRPS7, PRPS31, PRPL1), heat shock protein family members (PSSA1, PHSP90, PKAR2), 6-Phosphogluconate dehydrogenase (PGND1), phosphoglycerate mutase (PGPM1), transketolase (PTKL1), phosphatidylinositol synthase (PPIS1), ferro-O2-oxidoreductase (PFET3), high affinity iron permease (PFTR1), repressible alkaline phosphatise (PPHO8) N-myristoyl transferase (PNMT1), pheromone response transcription factor (PMCM1), ubiquitin (PUBI4), single-stranded DNA endonuclease (PRAD2) and the promoter of the major ADP/ATP carrier of the mitochondrial inner membrane (PPET9).

In a preferred expression system the promoter is an inducible or a constitutive promoter. The promoter can be an endogenous promoter or heterologous to the host cell.

The DNA sequence encoding the helper factor and/or the POI may also be operably connected to a suitable terminator sequence, for example AOX1 (alcohol oxidase) terminator, CYC1 (cytochrome c) terminator, TEF (translation elongation factor) terminator.

Expression vectors may comprise one or more phenotypic selectable markers, e.g. a gene encoding a protein that confers antibiotic resistance or that supplies an autotrophic requirement, and an origin of replication recognized by the intended host cell to ensure amplification within the host. Yeast vectors commonly contain an origin of replication from a yeast plasmid, an autonomously replicating sequence (ARS), or alternatively, a sequence used for integration into the host genome, a promoter region, sequences for polyadenylation, sequences for transcription termination, and a selectable marker.

The procedures used to ligate the DNA sequences, e.g. coding for the helper factor and/or the POI, the promoter and the terminator, respectively, and to insert them into suitable vectors containing the information necessary for integration or host replication, are well known to persons skilled in the art, e.g. described by J. Sambrook et al., "Molecular Cloning 2nd ed.", Cold Spring Harbor Laboratory Press (1989).

It will be understood that the vector, which uses the helper factor gene and/or the POI as an integration target, may be constructed either by first preparing a DNA construct containing the entire DNA sequence coding for the helper factor and/or the POI and subsequently inserting this fragment into a suitable expression vector, or by sequentially inserting DNA fragments containing genetic information for the individual elements, such as the signal, leader or heterologous protein, followed by ligation.

Also multicloning vectors, which are vectors having a multicloning site, can be used according to the invention, wherein a desired heterologous gene can be incorporated at a multicloning site to provide an expression vector. Expression vectors are vectors carrying a heterologous gene and used for expression of a heterologous compound encoded by such gene. In expression vectors, the promoter is placed upstream of the gene of the heterologous compound and regulates the expression of the gene. In the case of multicloning vectors, because the gene of the heterologous compound is introduced at the multicloning site, the promoter is placed upstream of the multicloning site.

The DNA construct as provided to obtain a recombinant host cell according to the invention may be prepared synthetically by established standard methods, e.g. the phosphoramidite method. The DNA construct may also be of genomic or cDNA origin, for instance obtained by preparing a genomic or cDNA library and screening for DNA sequences coding for all or part of the polypeptide of the invention by hybridization using synthetic oligonucleotide probes in accordance with standard techniques (Sambrook et al., Molecular Cloning: A Laboratorv Manual, Cold Spring Harbor, 1989). Finally, the DNA construct may be of mixed synthetic and genomic, mixed synthetic and cDNA or mixed genomic and cDNA origin prepared by annealing fragments of synthetic, genomic or cDNA origin, as appropriate, the fragments corresponding to various parts of the entire DNA construct, in accordance with standard techniques.

Transformants according to the present invention can be obtained by introducing such a vector DNA, e.g. plasmid DNA, into a host and selecting transformants which over-express the POI in the presence of a helper factor. Host cells are treated to enable them to incorporate foreign DNAs by methods conventionally used for transformation of eukaryotic cells, such as the electric pulse method, the protoplast method, the lithium acetate method, and modified methods thereof. *P. pastoris* is preferably transformed by electroporation.

In another preferred embodiment, the yeast expression vector is able to stably integrate in the yeast genome, e. g. by homologous recombination.

A transformant host cell according to the invention obtained by transforming the cell with the helper factor and/or the POI genes may preferably first be cultivated at conditions to grow efficiently to a large cell number without the burden of expressing a heterologous protein. When the cell line is prepared for the POI expression, cultivation techniques are chosen to produce the expression product.

When the transformant is grown with an inductive stimulus, a promoter may be activated to direct transcription of the gene under its control, and the POI is expressed. Under growth conditions with an inductive stimulus, it usually grows slower than under normal conditions, but because it has already grown to a large cell number in the previous stage, the culture system as a whole produces a large amount of the heterologous protein. An inductive stimulus is preferably heat, or addition of cadmium, copper, an osmotic pressure increasing agent, hydrogen peroxide, ethanol, methanol, methylamine or the like.

It is preferred to cultivate the host cell line according to the invention in a bioreactor under growth conditions to obtain a cell density of at least 1 g/L, more preferably at least 10 g/L cell dry weight, preferably at least 50 g/L cell dry weight. It is advantageous to provide for such yields of biomolecule production on a pilot or industrial scale.

The POI is preferably expressed employing conditions to produce yields of at least 1 mg/L, preferably at least 10 mg/L, preferably at least 100 mg/L, most preferred at least 1 g/L.

According to the present invention it is surprisingly possible to effectively co-express the helper factor and the POI with high yields, even when the biomass is kept low. Thus, a high specific yield, which is measured in mg POI/g dry biomass, in the range of 2 to 200, preferably 100 to 200, up to 500 in the pilot and industrial scale is thus feasible. The specific productivity of a production host according to the invention preferably provides for an increase of at least 1,1 fold, more preferably at least 1,5 fold, at least 1,2 or at least 1,3 fold, in some cases an increase of more than 2 fold can be shown, when compared to the expression of the product without the helper factors.

The host cell according to the invention is preferably tested for its expression capacity or yield by the following test: ELISA, activity assay, HPLC, or other suitable tests.

Preferred fermentation techniques are batch, fed batch or continuous cultivation.

Preferably the yeast is cultivated in a mineral medium with a suitable carbon source, avoiding a vitamin cocktail, thereby further simplifying the isolation process significantly. An example of a preferred mineral medium is one containing an utilizable carbon source (e.g. glucose, glycerol or methanol), salts containing the macro elements (potassium, magnesium, calcium, ammonium, chloride, sulphate, phosphate) and trace elements (copper, iodide, manganese, molybdate, cobalt, zinc, and iron salts, and boric acid).

The transformed cells are cultivated under conditions suitable to effect expression of the desired recombinant compound, which can be purified from the cells or culture medium, depending on the nature of the expression system and the expressed protein, e.g. whether the protein is fused to a signal peptide and whether the protein is soluble or membrane-bound. As will be understood by the skilled artisan, cultivation conditions will vary according to factors that include the type of host cell and particular expression vector employed.

If the desired compound is secreted from the cells, it can be isolated and purified from the culture medium using state of the art techniques. Secretion of the recombinant expression products from the yeast cells is generally advantageous for reasons that include facilitating the purification process, since the products are recovered from the culture supernatant rather than from the complex mixture of proteins that results when yeast cells are disrupted to release intracellular proteins.

Secreted proteins generally are initially expressed as precursors bearing an N-terminal signal or leader peptide. Signal peptides generally contain a positively charged N-terminus followed by a hydrophobic core, followed by a recognition site for an enzyme known as signal peptidase. This enzyme cleaves the signal peptide from the protein during translocation. The protein is transported from the endoplasmic reticulum to the Golgi apparatus, and then follows one of a number of routes in the secretory pathway, depending on the nature of the protein. The protein may be secreted into the culture medium or may be retained on the cell surface, for example. Certain receptors that comprise extracellular, transmembrane, and cytoplasmic domains are examples of proteins that may be retained on the cell membrane, with only the extracellular domain located outside the cell.

The cultured transformant cells may also be ruptured sonically or mechanically, enzymatically or chemically to obtain a cell extract containing the desired POI, from which the POI is isolated and purified.

As isolation and purification methods for obtaining a recombinant polypeptide or protein product, methods, such as methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used. Specific purification steps are preferably employed to separate any helper factor that is co-expressed and would contaminate the POI preparation.

The isolated and purified POI can be identified by conventional methods such as Western blotting or assay of its activity. The structure of the purified compound can be defined by amino acid analysis, amino-terminal analysis, primary structure analysis, and the like. It is preferred that the compound is obtainable in large amounts and in a high purity level, thus meeting the necessary requirements for being used as an active ingredient in pharmaceutical compositions.

The preferred host cell line according to the invention maintains the integration of the helper factor and POI genes, and the expression level remains high, e.g. at least at a µg level, even after about 20 generations of cultivation, preferably at least 30 generations, more preferably at least 40 generations, most preferred of at least 50 generations. The recombinant host cell is surprisingly stable, which is a great advantage when used for industrial scale protein production.

The present invention is described in further detail in the following examples, which are not in any way intended to limit the scope of the invention as claimed.

### Examples

### Example 1:

a) Construction of *P. pastoris* strain displaying Fab 2F5 on the surface
For the generation of the Fab 2F5 expression cassettes, the entire light chain genes (vL and cL) and the vH and cH1 region of the heavy chain genes were amplified by PCR from pRC/RSV containing the humanized IgG1 mAb (Kunert et al., 1998, 2000). Non template codes restriction sites were added by using oligonucleotide primer (Table 1)

**Table 1: Oligonucleotide primer for PCR amplification of Fab 2F5 heavy and light chain**

| | |
|---|---|
| 2F5 vH/hc FORW EcoRI | 5'-AGGAGAATTCAGGATCACGTTAAaGGAATC-3' (SEQ ID No. 5) |
| 2F5 vL/Ic FORW EcoRI | 5'-ACAAGAATTCGCCCTCCAACTGACCC-3' (SEQ ID No. 6) |
| cL BACK SacII | 5'-CCTTCCGCGGCTAACACTCTCCCCTG-3' (SEQ ID No. 7) |
| cH BACK EcoRI | 5'-GAATTCTTTGTCACAAGATTTGGGCTCAACTT-3' (SEQ ID No. 8) |

The light chain fragments were ligated into the EcoRI and SacII site of a modified version of pGAPalfaA (Invitrogen), where the AvrII restriction site was changed to a Ndel site by site directed mutagenesis to allow subsequent linearization of the plasmids containing two expression cassettes.
The heavy chain fragments were inserted into the EcoRI site of a modified version of pGAPZalfaA carrying alfa-agglutinin-fragment (alfa-Agglutinin fragment was amplified by PCR from *S.cerevisiae* genomic DNA using Primer Agg-EcorRI#1 (SEQ ID No. 9) and Agg-Xbal#2 (SEQ ID No. 10) (Table 2) and cloned into the EcoRI and Xbal site of pGAPZalfaA).

**Table 2: Oligonucleotide primer for PCR amplification of alfa-Agglutinin for S.cerevisiae**

| | |
|---|---|
| Agg-EcorRl#1 | 5'-AAGAATTCACAGCTAGCGCCAAAAGCTCTTTTATCT-3' (SEQ ID No. 9) |
| Agg-Xbal#2 | 5'-TGTTCTAGATTAGAATAGCAGGTACGACAA-3' (SEQ ID No. 10) |

A plasmid combining the expression cassettes for both Fab chains on one vector was produced by double digestion of the light chain vector with BgIII and BamHI, and subsequent insertion of this expression cassette into the unique BgIII site of the respective vector (pGAPZαA) already containing a single copy of the expression cassette of the heavy chain fragment fused to the alfa-agglutinin fragment.
To generate a plasmid conferring Geneticin (G418) resistance in *P. pastoris,* the *Sh ble* gene (conferring resistance to Zeocin (Zeocin™ is a trademark of CAYLA) of the plasmid pGAPZalfaA carrying the Fab 2F5 light chain and the heavy chain alfa-agglutinin fusion(pGAPZaLfaA-LC-HCaAggl) was replaced by the Tn903 kanR gene from the kanMX4 cassette of pFA6KanMX4 (Longtine et.al.1998). The exchange was done by cloning the Tn903 kanR gene (pFA6kanMX4 cut with Ncol and Scal) into the plasmid backbone of the pGAPZalfaA-LC-HCalfaAggl digested with Ncol and EcoRV.
The resulting plasmid was linearized with AvrII prior to electrotransformation into *P.pastoris* SMD1168 (Invitrogen).
To assure the display of the antibody fragment Fab 2F5 on the surface of *P. pastoris* flow cytometry analysis of immunofluorescent stained cells (with anti-human kappa light chain FITC conjugate; Sigma-Aldrich F3761) were performed and high producing (displaying) strains were selected.
b) Construction of the plasmid pLIB suitable for co-overexpression of a *P.*
*pastoris* cDNA library:
To generate pLIB in a first step the multiple cloning site (MCS) of pGAPZB (Invitrogen) was modified by inserting of an artificial oligonucleotide (annealed 5'phosphorelated oligonucleotide MCS#1 (SEQ ID No. 11) and MCS#2 (SEQ ID No. 12), (Table 3) coding for additional restriction sites BstAPI(A), Pacl and BstAPI(B) between the EcoRI and NotI site of pGAPZB. The restriction sites BstAPI(A) and BstAPI(B) were designed to form compatible sticky-ends with Sfil(A) / Sfil(B). In a second step the original GAP promoter was replaced by a PCR fragment of a modified GAP promoter, carrying two additional bp at position 238 (modified by PCR using the megaprimer method; oligonuceotide primer see Table 4) by cloning it between BgIII and EcoRI. The insertion of these two bp led to a novel Sfil restriction site.

**Table 3: Oligonucleotides for multiple cloning site**

| | |
|---|---|
| MCS #1 | 5'- PHO- AATTCTTAATTAAGCAGTTAATGCGGCGCGCCATGCAGCCTAT GCTTAATTAAGC-3' (SEQ ID No. 11) |
| MCS #2 | 5'- PHO- GGCCGCTTAATTAAGCATAGGCTGCATGGCGCGCCGCATTAA CTGCTTAATTAAG-3' (SEQ ID No. 12) |

**Table 4: Oligonucleotide primer for PCR amplification of a-Agglutinin for S.cerevisiae**

| | |
|---|---|
| Pgap-back-EcoRI | 5'- GTCGTGAATTCCTCGTTTCGAAATAGTTGTTCAATT-3' (SEQ ID No. 13) |
| Pgap-middle-Sfil | 5'- AAGAGCATTGCTGGCCAAGGGGGCCGTAGAAGAAGCTC TCC-3'(SEQ ID No. 14) |
| Pgap-for-BgIII | 5'- TGCATGAGATCAGATCTTTTTTGTAGAAATGT-3' (SEQ 10 No. 15) |

In another step the AOX1 transcription terminator and the Zeocin resistance cassette of the resulting plasmid pGAP(Sfil)MCSmod was changed against the CYC1 transcription terminator of S.cerevisiae - Zeocin resistance cassette fragment from the plasmid pPuzzie_ZeoR_Pgap_eGFP_AOXTT (Gasser et.al. 2008) To avoid gene replacement events (knockout of the expression cassettes of 2F5Fab heavy and/or 2F5Fab light chain) via homologous recombination the direction of the Zeocin resistence cassette was changed by restriction enzyme digest with KpnI and religation.
pPuzzle_ZeoR_Pgap_eGFP_AOXTT(reverse) was digested with BamHI (followed by treatment with Klenow-polymerase), and BssSI. The DNA fragment of the correct size was cloned into the plasmid pGAP(Sfil)MCSmod (digested with Agel, treated with Klenow and digested with BssSI). The resulting plasmid was called pLIB.
c) Construction of cDNA co-expression library:
cDNA synthesis was performed starting from polyA-RNA pool of different strains of *P. pastoris* grown under different environmental conditions (modified cultivation parameter: temperature, pH, different carbon and nitrogen sources; complex and minimal-salt media, heat shock) following the protocol of the SMART Creator cDNA Library Construction Kit (Clontech) until the step of cloning the cDNA PCR fragments. Instead of cloning the cDNA into the provided plasmid, the resulting cDNA-PCR fragments were cloned into the plasmid pLIB (example 1 step b), which was especially designed and constructed for this study (cDNA library was amplified by PCR and digested with SfII and cloned into the BstAPI and alkaline phosphatase treated pLIB). Transformation of *E*. *coli* via electrotransformation led to 1x10⁶ individual clones. The efficiency of the cloning procedure was checked by restriction enzyme analysis.

The resulting plasmid library (linearized with Sfil for integration into the genome via homologous recombination) was used to transform a pre-selected strain of *P. pastoris* displaying the antibody fragment Fab2F5 on the surface of the cell (example 1 step a). An optimized transformation procedure led to a *P. pastoris* cDNA co-expression library with 1.2x10⁶ individual clones on YPD agar containing Zeocin. All colonies were scraped off from the plates using YPD media. The resulting cell suspension was used to inoculate an expression culture with a starting optical density at 600nm of 0.1 (conditions: 100ml shake flask culture with 10ml BM media (per litre: 10 g yeast extract, 10 g peptone, 100 mM potassium phosphate buffer pH 6.0, 13.4 g yeast nitrogen base with ammonium sulfate, 0.4 mg biotin). Cells were grown at 28°C and 180 rpm to mid-log phase (OD600 8-15). About 10⁷ cell were immunofluorescent stained with anti-human-kappa-light-chain FITC conjugate (Sigma-Aldrich F3761, dilution 1:100) in PBS containing BSA and after 3 seconds of sonication they were used for fluorescent activated cell sorting (FACS). FACS was performed on a FACS Calibur (Becton Dickinson, Franklin Lakes, NJ, USA). For FACS two gates had to be defined. Gate 1 was defined on the dotplot FSC vs. SSC and it was used to distinguish cells and cells debris. Gate 2 was designed on a dotplot FL1 vs. FSC in that way that it collects only those cells of gate 1 with the highest FL1 signal. Only events (cells) belonging to both gates were sorted and collected in a cells concentrator. The ratio of sorted events vs. unsorted events (Table 5) was controlled via moving gate 2. The recovery of the sorted cells was done in liquid BM media. All recovered cells were used to inoculate a fresh expression culture and grown under above described conditions to mid-log phase again.

This procedure was repeated four times, so that a total of five consecutive rounds of sorting were performed.

**Table 5: Sorting rounds**

| sorting round | total events | sorted events | | Sorting mode |
|---|---|---|---|---|
| 1 | 1,007 x 10⁷ | 2.59 x 10⁵ | 2.59% | exclusion mode |
| 2 | 1.02 x 10⁷ | 1.46 x 10⁵ | 1.46% | exclusion mode |
| 3 | 1.50 x 10⁷ | 3.12 x 10⁴ | 0,207% | exclusion mode |
| 4 | 1.84 x 10⁷ | 1.76 x 10⁴ | 0,095% | exclusion mode |
| 5 | 2.10 x 10⁷ | 1.01 x 10⁴ | 0.048% | single cell |

After the 5^{th} round of expression, staining and sorting, all sorted cells were plated on YPD agar (per litre: 10g yeast extract, 20g peptone, 20 g glucose, 25mg Zeocin, 500mg G418-sulphate). After one round of replica plating, to individualize single clones, these single colonies were used to inoculate expression cultures and grown to mid-log phase again. An aliquot of cells from each individual clone was used for immunofluorescence staining followed by flow cytometry analysis. Flow cytometric analyses were performed on a FACS Calibur (Becton Dickinson, Franklin Lakes, NJ, USA). The probe was excited with a 488 nm air-cooled argon-ion laser while the fluorescence emission was measured through a 585/21 bandpass filter
All data were acquired in a logarithmic mode. Threshold settings were adjusted, so that the cell debris was excluded from the data acquisition. 10000 cells were measured for every sample. Data analysis was performed afterwards with FCSExpress V3 and MS Excel Software. The fluorescence emission of the immunofluorescence stained and surface-displayed antibody fragment 2F5Fab was normalized to the cell size, by calculation of ratio FL1/FSC. For each individual clone the geometric mean of FL1/FSC ratio of all 10000 measured cells was calculated and compared to the geometric mean of the FL1/FSC ration of a control strain not co-overexpressing a homologous gene. (*P.pastoris* 2F5Fab displaying strain transformed with an empty pLIB plasmid). Strains with an enhanced 2F5Fab cell surface signal were used for preparation of genomic DNA (DNeasy Blood and Tissue Kit, Qiagen 69504). Identification of co-overexpressed homologous genes was done by PCR amplification using pLIB specific oligonucleotide primer (GAPLibamp (SEQ ID No. 16) and Shble3out (SEQ ID No. 17), Table 6) and sequencing of the PCR product.

**Table 6: Oligonucleotide primer for PCR amplification and sequencing of co-overexpressed genes**

| | |
|---|---|
| GAPLibamp | 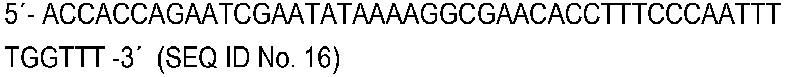 |
| Shble3out | 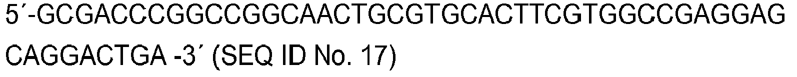 |

Sequences were blasted against *P.pastoris* genomic sequence, *S.cerevisiae* genome database and NCBI database. From the output of the 5^{th} sorting round three open reading frames of *P.pastoris* could be identified.

RPPA12105 *P. pastoris* IG-66. no functional annotation; preliminarily annotated as cLC52 (cDNA-library clone 52) Coding sequence (SEQ. ID No 1):

ATGAGTTTTCAAGTCTACAACATGGTGTCCTTGCCAGCACCGCAAGCAACATCATT
CTCCCAACATCCGAAACTTCCAACATTGGAAGTGATACAACGGCCACCACTCACT
GGAAGACATGGCATTCACGAAGAAGAGATCAAGCTACCTTCCTTTAGATCTTTACT
GGCTAGTGCGGGCACTGCCGCTGCTCTTGAATATCCCCCCAGGATTCAACCCCA
ATTTGCAACAGGGGCACCTGCACCTGAGCAAGCATTTTATCAGCCCCTAAATACC

GTGGGAGTCACGAGGCCCAACATTCACACCTCCATCACTCCACCACTGGAGGCG
CAGGACCTCCCTCACCAACAAAAGTTTATGGAACCATTTGCGTCCTTCCCCAATAC
AGTAGTGTCTTCTACACCCAACACAGCCATCAGAAAATACAAGTGCAAGATCTGC
GAGAGGTCTTTCACAACGTCGGGCCACTTAGCTCGTCACACACGTATTCACACTG
GTGAAAAGAAGCATGAGTGTCCTTTCGAAGGCTGTTCTGCTAGGTTCAGCCGTCA
GGATAACTGCATGCAACATTACAAGACGCATGTTAATACCAAGTCGAAACGAAAG
AGATCTAGATTGAGAAGTAAATAGGTA

RPPA10316 *P.pastoris* IG-66 Annotation: RPL33A by homology to S. *cerevisiae* Coding sequence (SEQ. ID No 2):

ATGGCTGAGGAAAACAGATTATACGTTAAGGGTAAGCACGTGTCTTTCCAACGTT
CCAAGAGTGTTATCCACCCAAAGACTTCCTTGATCAAAATTGAGGGTGTCGAAAA
CTCAAAGGATGCTGAGTTCTACATTGGTAAGAGAATCGCTTACGTCTACAAAGGT
GTAAAGGCTATCAACGGTACAAAGGTCAGAGTCATGTGGGGAACAGTCACCAGA
ACTCACGGAAACTCTGGTGTTGTCAGAGCTAAGTTCGAACGTAACCTTCCAGGTC
AGAGTTTTGGTTCCACTGTCAGAATCATGCTTTACCCATCTAACATCTAAATT

Genomic sequence including an intron (SEQ. ID No 3): 
ATGGCTGAGGAAAACAGATGTATGTACTATTAAATCGCAAGGTGACTGGTAAATG
ATGAAGATGATCGGCTTAATGAGAAGAATAATTGAAAACATAGCCAGAAAGTTTTG
ATTTCTGTTAAACCAAGCTGAAATGATGGGTTGGGAATTTTTTATTAAGCCAAATG
AACATTCTTACAACTTTAACCAACGGATATTATATATGGCTGAGCCAGCGTTATTC
CAAAAGCACAAGTGGGAACAGGTCTCTAAGTTTTTCTTTACTTTCAAGTTGAGTT
GACCGCTTCATCTTCATTTTCTCCGACTATCACCACCTTCTCACTATATTTTTAGA
TTCACGAGGTTACTAACTTTGCTATAGTATACGTTAAGGGTAAGCACGTGTCTTTC
CAACGTTCCAAGAGTGTTATCCACCCAAAGACTTCCTTGATCAAAATTGAGGGTGT
CGAAAACTCAAAGGATGCTGAGTTCTACATTGGTAAGAGAATCGCTTACGTCTACA
AAGGTGTAAAGGCTATCAACGGTACAAAGGTCAGAGTCATGTGGGGAACAGTCAC
CAGAACTCACGGAAACTCTGGTGTTGTCAGAGCTAAGTTCGAACGTAACCTTCCA
GGTCAGAGTTTTGGTTCCACTGTCAGAATCATGCTTTACCCATCTAACATCTAAAT
T
 RPPA09410 *P.pastoris* IG-66 no annotation (working name cLC61 cDNA-library clone 61) Coding sequence (SEQ. ID No 4):

ATGGCTCCCAGAACACTACCAGAAGACTTAATTCCCTCCCTATACGACTTGCACAT
CTACAACTTCCAACCCGAAAAAAGACTTATGATGGAGACATTGTCATCCACTTGG
AGGTGAAGGAGCCCACTGATGAAGTGGTCTTCAATGCCAAGGATTTGGAATTGAA
AGACGTACATGTCTTCCACAATGTCAACAAGTCTGAAAACGAAATCCCCGTTAAG
GAGATTGTTGATAACGAGCTCATCACAATTAAGCTCAAAGAGAAGGTTACTTCCG
GAACGTTGCTGGTGAATATTTCCTTCACCGGTAACATTCAATCTGATAAAATTGGA
TTTTACAAGGGAGACACAGATGTGGAAGGAAGAGTCACATACACTACAAACCTTA
CCACTCCAAATGCCAGGTTGGCATTCCCATGTCTTGATAACATATTGTTGAAAGCT
CCATTCAAGTTCGGAGTAACTGCCAATCCAGGACAATTAGTGAGTTCCATTTTGGA
TCTAAGCTCTGAGGCTGACGTCTTGAATGACAATGACGATGTGATTGGTACGAGA
TACCAATACCAAGTGAGTGAGCCAATAGCCCCAGCTTTACTGGAGTGGACCATTC
ATATTTAACGA

Identified open reading frames were amplified from pLIB cDNA-Library with specific oligonucleotide primers (Table 7) The *P. pastoris* Kozac sequence (GCC) was inserted directly before the start codon (ATG). The non template coded restrictions sites Sbfl and Sfil were added by using corresponding forward and backward primer. After treatment with the restriction enzymes Sbfl and Sfil the PCR products of the correct size were cloned into pPuzzle_ZeoR_Pgap_eGFP_AOXTT plasmid (Gasser et.al. 2008) replacing the open reading frame of eGFP (digested with Sbfl and Sfil followed by treatment with alkaline phosphatase). The resulting plasmids (pPZ-RPL; pPZ-cLC52, pPZ-cLC61) and an empty pPuzzle_ZeoR_Pgap_AOXTT were used to transform *P. pastoris* SMD1168 displaying 2F5Fab after restriction digest with Ascl. After one round of replica plating, to individualize single clones, single colonies were used to inoculate pre-cultures on YPD media. After incubation at 28°C at 180 rpm for about 20 hours these pre-cultures were used to inoculate an expression culture with OD600 = 0.1. Aliquots of expression cultures (grown to mid log phase at 28°C, 180 rpm) were used for immunfluorescence staining and flow cytometry analysis. Expression levels of different strains were compared by calculation of the geometric mean of FL1/FSC ratio (Table 8).

**Table 7: Oligonucleotide primer for PCR amplification of identified genes**

| | |
|---|---|
| RPL33_#1_Sbfl | 5´-GATTCACCTGCAGGCCATGGCTGAGGAAAACAGATTATAC GTTAAGGGT-3´ (SEQ ID No. 18) |
| RPL33_#2_Sfil | 5´-GGATGGCCGAGGCGGCCTTAGATGTTAGATGGGTAAAGC ATGATTCTG-3´ (SEQ ID No. 19) |
| cLC52_#1_Sbfl | 5´-GATTCACCTGCAGGCCATGAGTTTTCAAGTCTACAACATG GTGTCCTTGCCAG-3´ (SEQ ID No. 20) |
| cLC52_#2_Sfil | 5´-GGATGGCCGAGGCGGCCCTATTTACTTCTCAATCTAGATC TCTTTCGTTTCGAC-3´ (SEQ ID No. 21) |
| cLC61_#1_Sbfl | 5′-GATTCACCTGCAGGCCATGGCTCCCAGAACACTACCAGA AGACTTAATTCCCTCC-3′ (SEQ ID No. 22) |
| cLC61_#2_Sfil | 5′-GGATGGCCGAGGCGGCCTTAAATATGAATGGTCCACTCC AGTAAAGCTG-3′ (SEQ ID No. 23) |

**Table 8: Expression levels**

| Strain | Mean FL1/FSC ratio | fold change |
|---|---|---|
| Control | 0.2676 | 1 |
| RPL33A | 0.3205 | 1.20 |
| cLC52 | 0.4663 | 1.74 |
| cLC61 | 0.3889 | 1.45 |

### Example 2: Co-overexpression of novel helper factor genes in a strain of P.pastoris overexpressing recombinant human trypsinogen (rhTRP)

### a) Construction of co-overexpression plasmids

To generate a plasmid suitable for co-overexpression of homologous gene of *P.pastoris* in a strain already expressing a heterologous protein from a vector confirming resistance to Zeocin (see Example 2 step b), in a first step the KanMX4 cassette (confirming Geneticin resistance in *P. pastoris*) was amplified by PCR from pFA6kanMX4 plasmid (Longtine et.al.1998). Restriction sites for KpnI were added by using specific oligonucleotide primer KanR#1 (SEQ ID No. 24) and KanR#2 (SEQ ID No. 25) (Table 9). The KpnI treated PCR fragment was cloned instead of the Zeocin resistance cassette between both KpnI sites of pPuzzle_ZeoR_Pgap_eGFP_AOXTT (Gasser et.al. 2008). The resulting vector was called pPkanR.

**Table 9:**

| | |
|---|---|
| KanR#1 | 5'-CCGAGGTACCGACATGGAGGCCCAGAATA-3' (SEQ ID No. 24) |
| Kan R#2 | 5'-CCGAGGTACCAGTATAGCGACCAGCATTCA-3' (SEQ ID No. 25) |

All genes of interest for co-overexpression (see Example 1) were amplified via PCR from *P. pastoris* cDNA library. Non template coded *P. pastoris* Kozak sequence and restriction sites for Sbfl and Sfil were added by using respective forward and backward oligonucleotide primer (Table 7). Sbfi and Sfil treated PCR products were cloned into pPKanR (Sbfl and Sfil digested and treated with alkaline phosphatase).

Novel co-overexpression plasmids pPKanR-RPL, pPKanR-cLC52 and pPKanR-cLC61 were transformed into E.coli Top10 (Invitrogen). Restriction endonuclease digest and sequencing was done to verify the correct identity of the constructed plasmid.

### b) Construction of P. pastoris strains co-overexpressing recombinant human trypsinogen and a novel helper factor gene

The plasmids pPKanR-RPL, pPKanR-cLC52 and pPKanR-cLC61, obtained from cloning procedure described in Example 2 step a) were used to transform a strain of *P. pastoris* pre-selected for the high level expression of recombinant human trypsinogen under the control of the GAP promoter (Hohenblum et.at 2004). Selection was based on Zeocin resistance for the trypsinogen gene and Geneticin resistance for the helper factor genes.
To evaluate the effect of co-overexpressed helper factor gene the trypsinogen expressing strain was also transformed with a pPkanR plasmid without helper factor gene.

### c) Culturing transformed P. pastoris strains in shake flask cultures

5 ml YP-medium (10 g/l yeast extract, 20 g/l peptone) containing 10 g/l glycerol were inoculated with a single colony of *P. pastoris* strains from step a) Example 3 and grown overnight at 28°C. Aliquots of these cultures (corresponding to a final OD600 of 0.1) were transferred to 12,5 ml of expressions culture medium (per liter: 10 g yeast extract, 10 g pea-peptone, 10,2g (NH4)2PO4, 1,24g KCI, 0,1g CaCl2, pH 5,0 adjusted with HCl) supplemented with 20g/L glucose and incubated for 60 h at 28°C C at vigorous shaking in 100 ml Erlenmeyer flasks. The same amounts of substrate were added repeatedly 4 times every 12 h, before cells were harvested by centrifugation at 2500xg for 10 min at room temperature and prepared for analysis (biomass determination by measuring the cell weight of 1 ml cell suspension), enzyme activity assay for trypsinogen quantification in the culture supernatant).

### d) Evaluation of the effect of co-overexpression of novel helper factors by quantification of rhTRP

Desalting of *P. pastoris* culture supernatant was done using small pre-packed size exclusions chromatography columns (Disposable PD-10 Desalting Columns 17-0851-01; GE Healthcare) 2,5 ml of supernatant were applied to the column and eluted with 3,5ml of elution buffer (1 mM HCl). After elution 70µl of 2M CaCl2 solution was added. To convert inactive trypsinogen to the active trypsin 300µl of the desalted supernatant (+CaCl2) were mixed with 690µl of activation buffer (50mM TRIS/HCl pH 8,6; 40mM CaCl2 0,15g/L Enterokinase from porcine intestine Sigma-Aldrich E0632) and incubated for two hours at 37°C. 165µl of the activation mixture was mixed with 1000µl of *N*_{α}-*p*-Tosyl-L-arginine-methyl-ester-hydrochloride (TAME) solution, containing 446 mg/L TAME (Sigma-Aldtrich T4626) dissolved in dilution buffer (50 mM TRIS/HCl pH 8,1; 40mM CaCl2) and an absorption kinetic at 247nm was measured in a spectrophotometer over a time period of 5 min. If necessary, activated trypsin solution was diluted with dilution buffer to hit the linear range (ΔA₂₄₇/min < 0.3 ) of this method. A trypsin concentration of 1g/L corresponds to ΔA₂₄₇/min= 0.101.

The evaluation of the data showed that the co-overexpression of PpcLC52, PpRPL33A and PpcLC61 had a significant positive effect on the amount of secreted recombinant human trypsinogen (Table 10).

### Example 3: Co-overexpression of the novel helper factor PpcLC52 in a P.pastoris strain expressing Fab fragment of the anti-HIV1 antibody 2F5

### a) Construction of the P. pastoris strain SMD 1168 secreting the Fab fragment of the monoclonal anti-HIV1 antibody 2F5.

Plasmids combining the expression cassettes for both Fab chains on one vector were produced by double digestion of the light chain vector with BgIII and BamHI, and subsequent insertion into the unique BamHI of the vector pGAPZalfaA already containing a single copy of the expression cassette of the heavy chain fragment. Plasmids were then linearized with AvrII prior to electrotransformation into *P. pastoris.*
All constructed expression cassettes were checked by DNA sequencing with the GAP forw/AOX3' backw (Invitrogen)
The resulting plasmid was used to transform *P. pastoris* SMD1168 (Invitrogen) after restriction digest with Ndel. Transformants were selected for Zeocin resistance and screened for high level expression of the Fab fragment of the monoclonal anti-HIV1 antibody 2F5.

### b) Construction of a P. pastoris strain co-overexpressing 2F5 Fab and the helper factor PpcLC52:

Transformation of a *P. pastoris* strain obtained in step a) Example 3 was carried out with the plasmid pKanR-cLC52 of step a) Example 2, which was linearized with Ascl. The plasmid was introduced into the cells by electrotransformation. The transformed cells were cultivated on YP-agar containing 20g/L glucose and 500mg/L Geneticin. As a control, the 2F5 Fab expressing strain was also transformed with a pPkanR plasmid without helper factor gene.

### c) Culturing transformed P. pastoris strains in shake flask cultures

5 ml YP-medium (10 g/l yeast extract, 20 g/l peptone) containing 10 g/l glycerol were inoculated with a single colony of *P. pastoris* transformants from b) Example 3 and grown overnight at 28°C. Aliquots of these cultures (corresponding to a final OD600 = 0.1) were transferred to 10 ml of expressions culture medium (per liter: 10 g yeast extract, 10 g peptone, 100 mM potassium phosphate buffer pH 6.0, 13.4g yeast nitrogen base with ammonium sulfate, 0.4 mg biotin) supplemented with 20g/l glucose and incubated for 48 h at 28°C C at vigorous shaking in 100 ml Erlenmeyer flasks. The same amounts of substrate were added repeatedly 3 times every 12 h, before cells were harvested by centrifugation at 2500xg for 5 min at room temperature and prepared for analysis (biomass determination by measuring the cell weight of 1 ml cell suspension, ELISA for Fab quantification in the culture supernatant).

### d) Evaluation of the effect of co-expression helper factors by quantification of 2F5Fab

To determine the amount of secreted recombinant expressed 2F5 Fab, 96 well microtiter plates (MaxiSorb, Nunc, Denmark) were coated with anti-human IgG (Fab specific) overnight at RT (Sigma-Aldrich 15260; 1:1000 in PBS, pH 7.4), before serially diluted supernatants of *P. pastoris* cultures secreting 2F5 Fab from step d) (starting with a 1:50 dilution in PBS/Tween20 (0.1 %) + 2 % BSA) were applied and incubated for 2 h at RT. A human Fab fragment of IgG (Rockland) was used as a standard protein at a starting concentration of 200 ng/ml. After each incubation step the plates were washed three times with PBS containing 0.1 % Tween 20 (adjusted to pH 7.4). 100 µl of anti-human-kappa light chain - AP conjugate as secondary antibody (Sigma-Aldrich A-3813 1:1000 in PBS/Tween + 2 % BSA) were added to each well, and incubated for 1 h at RT. After washing, plates were stained with pNPP ,1 mg/ml p-nitrophenyl phosphate in detection buffer, (0.1 N Na₂CO₃/NaHCO₃ pH 9.6) and read at 405 nm (reference wavelength 620 nm). The data are summarized in Table 10.

### Example 4:

### a) Construction of a P.pastoris strain overexpressing recombinant porcine trypsinogen.

For expression of recombinant porcine trypsinogen (rpTRP) a codon-optimized artificial gene was synthesized (Geneart Germany) During DNA synthesis a *P. pastoris* Kozak sequence (GCC just before the start codon ATG), the S. *cerevisiae* mating factor alfa secretion leader sequence, and sites for the restriction enzymes Sbfl and Sfil, flanking the open reading frame were added. The coding sequence of the rpTRP gene (flanked by the Kozak sequence) was cut out of the delivered plasmid using Sbfl and Sfil, and cloned into a Sbfl, Sfil and alkaline phophatase treated plasmid pPuzzle_ZeoR_Pgap_eGFP_AOXTT (Gasser et.at 2008) replacing the open reading frame of the eGFP gene. The ligated plasmid was transformed into *E.coli* TOP1 0 (Invitrogen) and plated on Zeocin containing LB-agar. Restriction endonuclease analysis was performed to confirm the correct identity of the plasmid pPZ_Pgap_rpTRP_AOXTT. After linearization with Ascl the vector was used to transform the *P.pastoris* strain X33 (Invitrogen) and plated on Zeocin and glucose containing YP- agar.

### b) Construction of P. pastoris strains co-overexpression rpTRP and the novel helper factor gene PpcLC52

The plasmid pPKanR-cLC52 obtained from cloning procedure described in Example 2 a) were used to transform a strain of *P.pastoris* pre-selected for the high level expression of rpTRP under the control of the GAP promoter, as described in Example 4 a). Selection was based on Zeocin resistance for the rpTRP gene and Geneticin resistance for the helper factor gene. To evaluate the effect of co-overexpressed helper factor genes on the expression of rpTRP, the trypsinogen expressing strain was also transformed with a pPkanR plasmid without helper factor gene.

### c) Culturing transformed P. pastoris strains in shake flask cultures

Cultivations of *P. pastoris* strains obtained from Example 3 step b) were done as described in Example 2 step c) with little modification. Instead of measuring the wet cell weight, the yeast dry mass of 10 ml cell suspension was appointed to determine the produced amount of biomass.

### d) Evaluation of the effect of co-overexpression of the helper factor by quantification of rhTRP

Determination of the amount of recombinant porcine trypsinogen was performed as described in Example 3 step d for recombinant human trypsinogen). The data are shown in Table 10.

**TABLE 10**

| **product** | **coexpressed gene** | **Product concentration** | **fold change product** | **fold change product/biomass** |
|---|---|---|---|---|
| rhTRP | RPL33A | 5.08 | 1.24 | 1.26 |
| rhTRP | cLC52 | 7.49 | 1.84 | 1.80 |
| rhTRP | cLC61 | 4.66 | 1.14 | 1.19 |
| rhTRP | control | 4.08 | -- | -- |
| 2F5 | cLC52 | 6.65 | 1.26 | 1.83 |
| 2F5 | control | 5.27 | -- | -- |
| rpTrp | cLC52 | 37.91 | 1.41 | 1.34 |
| rpTrp | control | 26.87 | -- | -- |

### Example 5: Cultivation of a P. pastoris strain co-overexpressing porcine trypsinogen and PpcLC52 in fermentation cultures

### Batch-Fermentation of P. pastoris

Fermentations were performed in a bioreactor, which is a pilot scale for industrial production, with a total volume of 5.0 I (Minifors, Infors, Switzerland) with a computer-based process control system. The media were as follows:
All chemicals for PTM₁ trace salts stock solution were from Riedel-de Haën, except for biotin (Sigma), and H₂SO₄ (Merck Eurolab).
PTM1 trace salts stock solution contained per litre: 6.0 g CuSO₄.5H₂O, 0.08 g Nal, 3.0 g MnSO₄. H₂O, 0.2 g Na₂MoO₄. 2H₂O, 0.02 g H₃BO₃, 0.5 g CoCl₂, 20.0 g ZnCl₂, 65.0 g FeSO₄. 7H₂O, 0.2 g biotin and 5.0 ml H₂SO₄ (95 %-98 %).
Batch medium contained per litre: 2.0g/L Citric-acid xH₂O, 12.6 g (NH₄)₂HPO₄, 0.022 g CaCl₂ x2H₂O, 0.9 g KCI, 0.5 g MgSO₄ x7H₂O, 40 g glycerol, 4.6 ml PTM1 trace salts stock solution and 0.4mg D-Biotin. The pH was adjusted to 5.0 with 25% HCl.
The dissolved-oxygen concentration was maintained above 20% saturation by controlling the stirrer speed between 600 and 1250 rpm. Aeration rate was 120 L* h⁻¹ air. The temperature was 25°C, and the pH was controlled with NH₃ (25 %). Before starting the fermentation, the pH of the batch medium was set to 5.0.
The batch phase of approximately 24 to 30 h led to a dry biomass concentration of approximately 20 g I⁻¹. In the feed media glucose (composition depending on feed strategy, see step b), step c) and step d)) serves as a carbon source. The pH was 5.0 during batch, and kept at 5.0 throughout the fermentation.
Samples were taken at the end of the glycerol batch phase, during and/or at the end of the feeding phase. 10ml of cell suspension were centrifuged to separate the cells from culture supernatant, then the cell pellets were washed with water and dried at 105°C for 24h to determine cell dry weight (YDM). The supernatant was frozen at - 20°C and keep until trypsinogen assay (as described for human trypsinogen step c example 3).

### a) Strains

A strain obtained from Example 4 step b),co-overexpressing recombinant porcine trypsinogen and PpcLC52, and the "wildtyp" strain were used to inoculate a preculture on YPG (10 g/L yeast extract, 20 g/L peptone, 10 g/L glycerol). Precultures were incubated with shaking at 28°C for 24h. To remove complex media components cells were harvested by centrifugation at 1500 x g for 10 min and re-suspended in batch media. Resulting cells suspension was used to inoculate the initial batch phase at a starting optical density at 600 nm of 1.0

### b) Chemostat-Fermentation of a P. pastoris strain co-overexpressing rpTRP and PpcLC52.

After an initial batch phase (batch media volume 1.75 litre) the fermentation was run in a chemostat mode with a specific growth rate of µ=0.1 and a biomass concentration (yeast dry mass) of 25 g/L. Feed-media flow (1.0g/L Citric-acid xH₂O; 4.4g/L (NH₄)₂HPO₄, 0.01 g/L CaCl₂ x2H₂O, 1.7g/L KCI, 0.7 g/L MgSO₄ x7H₂O, 55 g/L glucose xH₂O, 1.6 ml/L PTM1 trace salts stock solution and 0.4mg/L D-biotin; pH was adjusted to 5.0 with 25% HCl) and cell suspension harvest flow were set to 175 g/h. Air flow was keep constant a 120 L/h. Samples were taken at the end of the batch phase and after 5 volume changes (50h feeding).

On glucose as single carbon source and steady-state condition with a specific growth rate of 0.1 h⁻¹ the product concentration and the specific productivity of the PpcLC52 co-overexpressing strain was 1.4 fold higher compared to the control strain.

### c) Fedbatch-Fermentation of a P. pastoris strain co-overexpressing rpTRP and PpcLC52 with linear feeding.

After an initial batch phase (batch media volume 1.6 litre) the fermentation was run in a Fedbatch mode with a linear feeding profile. Feed-media flow (0.28 g/L CaCl₂ x2H₂O, 8.4g/L KCI 5.2 g MgSO₄ x7H₂O, 464 g/L glucose x H2O, 10.0 ml/L PTM1 trace salts stock solution and 0.4 mg/L D-Biotin) was set to 17.5 g/h. Air flow was keep constant a 120 L/h. Samples were taken after the batch phase, every 24h during the feeding period and at the end of feeding (100h).

The comparison of PpcLC52 co-overexpressing strain (rpTRP_cLC52) and the non co-overexpression strain (rpTRP_wt) showed an equal (linear) accumulation of biomass. The average specific productivity of the rpTRP_cLC52 strain was 1.3 fold higher, and the total product amount accumulated with rpTRP_cLC52 was 1.2 higher compared to rpTRP_control.

### c) Fed-batch fermentation of a P.pastoris strain co-overexpressing porcine trypsinogen and PpcLC52 with constant specific growth rate of µ=0.1 h⁻¹

After an initial batch phase (batch media volume 1.4 litres) the fermentation was run in a fed-batch mode with exponential feeding profile. Feed-media flow (0.28 g/L CaCl₂ x2H₂O, 8.4g/L KCI 5.2 g MgSO₄ x7H₂O, 464 g/L glucose xH₂O, 10.0 ml/L PTM1 trace salts stock solution and 0.4 mg/L D-biotin) was adjusted by a PID controller to keep a constant specific growth rate of µ=0.1 h⁻¹. The dissolved-oxygen concentration was maintained at 20% saturation by controlling the stirrer speed between 600 and 1250 rpm and the air flow between 120 and 500 L*h⁻¹. Due to limitation in oxygen transfer rate of the reactor the process ran in oxygen limitation after 16h of feeding. Samples were taken after the batch phase, 6 times during the feeding period and at the end of feeding (approximately. 25h)

The comparison of PpcLC52 co-overexpressing strain (rpTRP_cLC52) and the non co-overexpression strain (rpTRP_wt) showed an equal accumulation of biomass. The total product amount (cell free culture volume * product concentration) of the PpcLC52 co-overexpressing strain was 1.2 fold higher at the end of the fed batch phase compared to the control strain. The specific productivity of the rpTRP_cLC52 strain was up to 2.2 fold higher.

### Example 6: Construction of wildcard strains:

The plasmids pPKanR-RPL, pPKanR-cLC52 and pPKanR-cLC61, obtained from cloning procedure described in Example 2 step a) are used to transform a strain of *P. pastoris* which does not express a POI. Selection is based on Geneticin resistance for the helper factor genes. These transformed strains can further be used as improved production strains for POIs.

### References

Archer, D., Jeenes, D. and Mackenzie, D. (1994). Strategies for improving heterologous protein production from filamentous fungi. Antonie Van Leeuwenhoek 65, 245-50.
Gething, M. and Sambrook, J. (1992). Protein folding in the cell. Nature 355, 33-45.
Hohenblum, H., et al. (2003) Assessing viability and cell-associated product of recombinant protein producing Pichia pastoris with flow cytometry. J Biotechnol 102, 281-290
Kurtzman CP. (2005). Description of Komagataella phaffii sp. nov. and the transfer of Pichia pseudopastoris to the methylotrophic yeast genus Komagataella. Int J Syst Evol Microbiol 55, 973-976.
Lang, C. and Looman, A. (1995). Efficient expression and secretion of Aspergillus niger RH5344 polygalacturonase in Saccharomyces cerevisiae. Appl Microbiol Biotechnol 44, 147-56.
Macauley-Patrick, S., Fazenda, M. L., McNeil, B. and Harvey, L. M. (2005). Heterologous protein production using the Pichia pastoris expression system. Yeast 22, 249-70.
Mattanovich, D., Gasser, B., Hohenblum, H. and Sauer, M. (2004). Stress in recombinant protein producing yeasts. J Biotechnol 113, 121-35.
Mori, K., Ogawa, N., Kawahara, T., Yanagi, H. and Yura, T. (2000). mRNA splicing-mediated C-terminal replacement of transcription factor Hac1p is required for efficient activation of the unfolded protein response. Proc Natl Acad Sci U S A 97, 4660-5.
Porro, D., Sauer, M., Branduardi, P. and Mattanovich, D. (2005). Recombinant protein production in yeasts. Mol Biotechnol 31, 245-59.
Punt, P. J., van Biezen, N., Conesa, A., Albers, A., Mangnus, J. and van den Hondel, C. (2002). Filamentous fungi as cell factories for heterologous protein production. Trends Biotechnol 20, 200-6.
Sauer, M., Branduardi, P., Gasser, B., Valli, M., Maurer, M., Porro, D. and Mattanovich, D. (2004). Differential gene expression in recombinant Pichia pastoris analysed by heterologous DNA microarray hybridisation. Microb Cell Fact 3, 17.
Shuster, J. (1991). Gene expression in yeast: protein secretion. Curr Opin Biotechnol 2, 685-90.
Stryer, L. (1995). Biochemie. Spektrum der Wissenschaft Verlags GmbH. LONGTINE, M. S., A. MCKENZIE, 3RD, D. J. DEMARINI, N. G. SHAH, A. WACH et al., 1998 Additional modules for versatile and economical PCR-based gene deletion and modification in Saccharomyces cerevisiae. Yeast 14: 953-961 Hohenblum H, Gasser B, Maurer M, Borth N, Mattanovich D. Effects of Gene Dosage, Promoters, and Substrates on Unfolded Protein Stress of Recombinant Pichia pastoris Biotechnol Bioeng. 2004 Feb 20;85(4):367-75.
Gasser, B., Maurer, M., Gach, J., Kunert, R. and Mattanovich, D. (2006). Engineering of Pichia pastoris for improved production of antibody fragments. Biotechnol Bioeng 94, 353-61.
Gasser, B., Mattanovich, D., Sauer, M., Stadlmayr, G. (2008) WO 2008/128701A2 Expression System

## Claims

1. Eukaryotic host cell comprising a recombinant nucleotide sequence encoding an expression enhancer, which is selected from the group consisting of cLC52, RPL33 and cLC61.

2. Host cell according to claim 1, wherein said nucleotide sequence is originating from P. pastoris.

3. Host cell according to claim 1 or 2, which is a fungal cell, preferably a yeast cell, or a higher eukaryotic cell, preferably a mammalian or a plant cell.

4. Host cell according to claim 3, wherein the yeast cell is a cell of the *Pichia* genus, in particular a cell of a strain of *P. pastoris.*

5. Host cell according to any one of claims 1 to 4, which is a wildcard host cell for the introduction of a gene of interest encoding a protein of interest (POI).

6. Host cell according to any one of claims 1 to 4, which is a producer cell comprising a recombinant nucleotide sequence encoding a POI, capable of producing the POI.

7. *P. pastoris* host cell comprising
- a recombinant nucleotide sequence encoding an expression enhancer, which is selected from the group consisting of cLC52, RPL33, cLC61 and functional homologues thereof, and
- a gene of interest encoding a POI.

8. The use of a nucleotide sequence encoding a protein selected from the group consisting of cLC52, RPL33 cLC61 and functional homologues thereof, as an expression enhancer to increase the expression of a POI in a eukaryotic host cell.

9. A method of producing a host cell according to claim 6, comprising
- providing a host cell according to claim 5, and
- introducing a nucleotide sequence encoding a POI.

10. A method of producing a host cell according to claim 6, comprising
- providing a eukaryotic host cell,
- introducing a nucleotide sequence encoding at least one of cLC52, RPL33, cLC61 and functional homologues thereof, and
- introducing a nucleotide sequence encoding a POI.

11. A method of producing a POI in a eukaryotic cell, comprising:
- providing a producer host cell according to claim 6,
- co-expressing the POI and the expression enhancer in a cell culture, and
- obtaining the POI from the cell culture.

12. A method according to claim 11, wherein said POI is a polypeptide selected from the group consisting of serum proteins, such as an immunoglobulin or serum albumin, enzymes, hormones, signalling molecules, matrix proteins, fragments or derivatives thereof.

13. A method according to claim 11, wherein said POI mediates the production of a host cell metabolite.

14. A method according to any of claim 11 or 12, wherein the recombinant nucleotide sequence encoding a POI is provided on a plasmid suitable for integration into the genome of the host cell or for autonomous replication in the host cell.

15. A method according to claim 13, wherein the plasmid is a eukaryotic expression vector, preferably a yeast expression vector comprising a secretion leader sequence effective to cause secretion of the POI from the host cell and/or a promoter sequence effective to cause expression of the POI in the host cell.

16. Co-overexpression plasmid for use in a method according to any of claims 10 to 14 comprising
- the recombinant nucleotide sequence encoding a POI, and
- a nucleotide sequence encoding a protein selected from the group consisting of cLC52, RPL33 cLC61 and functional homologues thereof.
